Europäisches Patentamt

European Patent Office

Office européen des brevets

(11) Veröffentlichungsnummer: **0 337 083**
**A2**

(12) **EUROPÄISCHE PATENTANMELDUNG**

(21) Anmeldenummer: 89102946.4

(51) Int. Cl.⁴: **C07C 69/533 , C07C 67/38**

(22) Anmeldetag: 21.02.89

(30) Priorität: 13.04.88 DE 3812184

(43) Veröffentlichungstag der Anmeldung:
18.10.89 Patentblatt 89/42

(84) Benannte Vertragsstaaten:
**BE DE FR GB IT NL**

(71) Anmelder: **HÜLS AKTIENGESELLSCHAFT**
**Patentabteilung / PB 15 - Postfach 13 20**
**D-4370 Marl 1(DE)**

(72) Erfinder: **Gull, Reinhold, Dr.**
**Leverkusener Strasse 29**
**D-4370 Marl(DE)**
Erfinder: **Keil, Thomas, Dr.**
**Dormagener Strasse 36**
**D-4370 Marl(DE)**

(54) Verfahren zur Herstellung von w-en-Carbonsäureestern.

(57) $\omega$-en-Carbonsäuren können bei der Wolff-Kishner-Reduktion von $\omega$-en-Ketocarbonsäuren erhalten werden. Einige $\omega$-en-Carbonsäureester sind durch Spaltung von Lactonen herstellbar.

Erfindungsgemäß werden nun $\omega$-en-Carbonsäureester aus $\alpha,\omega$-Dienen durch Hydrocarboxyalkylierung mit Kohlenmonoxid und Wasserstoff mit Hilfe eines Kobaltkatalysators und eines Amin als Promotor in einem Alkohol hergestellt.

Herstellung von $\omega$-en-Carbonsäureestern.

EP 0 337 083 A2

## Verfahren zur Herstellung von ω-en-Carbonsäureestern

Die Erfindung betrifft ein Verfahren zur Herstellung von ω-en-Carbonsäureestern durch Hydrocarboxyalkylierung von α,ω-Dienen.

ω-en-Carbonsäurester sind wertvolle Zwischenprodukte zur Herstellung von Waschmitteln, Schmiermitteln, Emulgatoren, Weichmachern, Alkydharzen, Polyamiden sowie von Riech- und Geschmacksstoffen.

ω-en-Carbonsäuren mit bis zu 13 C-Atomen können nach A. Seher, Fette-Seifen-Anstrichmittel 58 (1956), 1077 aus kleinen Bausteinen in mehreren Schritten synthetisiert werden. Nach S. Hünig und W. Eckardt, Chemische Berichte 95 (1962), 2498 können ω-en-Carbonsäuren aus ω-en-Ketocarbonsäuren durch Wolff-Kishner-Reduktion hergestellt werden. Die Ausgangsverbindungen dieser Reaktion sind nicht in technischen Mengen verfügbar. Außerdem wird bei der Reduktion die Doppelbindung teilweise isomerisiert. Beide Methoden haben keine technische Bedeutung erlangt.

Nach DE-A 36 09 138 und DE-A 36 09 139 können ω-en-Carbonsäureester mit Ketten mit 5 bis 7 C-Atomen durch Spaltung von Lactonen erhalten werden. Bei dieser Methode, die nur für die Herstellung kurzkettiger ω-en-Carbonsäureester geeignet ist, wird die Doppelbindung zum Teil isomerisiert. Dadurch werden auch Produkte mit innenständiger Doppelbindung in erheblichen Mengen synthetisiert.

Nach DE-A 30 24 884 können ungesättigte $C_9$-Carbonsäuren durch carboxylierende Dimerisation von 1,3-Butadien mit Kohlendioxid in Gegenwart von Palladium-Verbindungen, Phosphin und Ameisensäure hergestellt werden. Die im Gemisch erhaltene 2-Ethylidenhept-6-encarbonsäure weist jedoch neben einer endständigen auch eine innenständige Doppelbindung und eine Verzweigung auf.

Nach J. Falbe, Synthesen mit Kohlenmonoxid, 1967, 102 können Olefine in Gegenwart von Alkoholen mit Kohlenmonoxid hydrocarboxyalkyliert werden. Aus 1,5-Cyclooctadien werden so an Pd/HCl ungesättigte Mono carbonsäureester hergestellt, während an Kobaltkatalysatoren stets die gesättigten Monocarbonsäuren entstehen. 1,5-Hexadien reagiert an einem Palladiumkatalysator unter Cyclisierung.

Ein Verfahren zur Hydrocarboxyalkylierung von α,ω-Dienen unter Bildung von ω-en-Carbonsäureestern wird von J. Falbe nicht beschrieben.

Aufgabe der vorliegenden Erfindung ist es, weitgehend unverzweigte ω-en-Carbonsäureester aus preiswerten, einfachen und in großen Mengen zugänglichen Ausgangsverbindungen herzustellen.

Diese Aufgabe wird erfindungsgemäß dadurch gelöst, daß man α,ω-Diene mit 5 bis 20 C-Atomen mit Kohlenmonoxid in Gegenwart einer Kobaltverbindung und eines tertiären Amins in einem Alkohol bei 100 bis 200 °C und 150 bis 350 bar hydrocarboxyalkyliert.

Diese Lösung ist überraschend. Denn die α,ω-Diene und ω-en-Carbonsäureester besitzen Doppelbindungen mit etwa der gleichen Reaktivität.

Für die Hydrocarboxyalkylierung werden vorzugsweise α,ω-Diene mit 8 bis 16 C-Atomen eingesetzt.

Eine geeignete Kobaltverbindung ist Kobalttracarbonylwasserstoff $HCo(CO)_4$. Es können auch Kobaltsalze wie Kobalt-(II)-acetat, -naphthenat, -stearat, -carbonat oder -chlorid, Kobaltoxide oder Kobaltcarbonyle wie Dikobaltoctacarbonyl eingesetzt werden. Diese Kobaltverbindungen erfordern in der Anfangsphase der Hydrocarboxyalkylierung neben Kohlenmonoxid 0,1 bis 10 Mol-% Wasserstoff, bezogen auf Mole Kohlenmonoxid. Dabei wird unter den gegebenen Reaktionsbedingungen von 100 bis 200 °C und 150 bis 350 bar Kobälttetracarbonylwasserstoff gebildet, das vermutlich die eigentlich katalytisch aktive Verbindung ist. Die Bildung des Kobalttetracarbonylwasserstoffs ist im allgemeinen nach einer halben Stunde beendet.

Bezogen auf das eingesetzte α,ω-Dien verwendet man vorzugsweise 0,5 bis 5 Mol-% Kobaltverbindung.

Als tertiäres Amin, das als Promotor wirkt, kommen Pyridin und nicht-ortho-alkylierte Pyridine wie zum Beispiel 3- und 4-Picolin, 3,4- und 3,5-Lutidin und 3- und 4-Ethylpyridin infrage. Vorzugsweise werden Pyridin, 4-Picolin und Gemische davon eingesetzt. Das Molverhältnis tertiäres Amin : Kobaltverbindung beträgt vorzugsweise 2 : 1 bis 300 : 1.

Als Lösemittel und Reagenz verwendet man im allgemeinen Alkohole mit 1 bis 6 C-Atomen wie Methanol, Ethanol, Isopropanol, Butanol oder Hexanol. Methanol ist dabei bevorzugt. Vorzugsweise stellt man ein Molverhältnis α,ω-Dien : Alkohol von 1 : 1 bis 1 : 4 ein.

Die Reaktion wird im allgemeinen in einem Autoklaven durchgeführt. Dabei wird vorzugsweise eine Temperatur von 120 bis 160 °C und ein Druck von 200 bis 300 bar eingestellt.

Durch das erfindungsgemäße Verfahren wird ein allgemeiner Weg dafür aufgezeigt, wie ω-en-Carbonsäureester aus α,ω-Dienen durch Hydrocarboxyalkylierung hergestellt werden können. Obwohl Ausgangs- und Endprodukte Doppelbindungen mit etwa der gleichen Reaktivität besitzen, werden ω-en-Carbonsäureester mit hoher Selektivität und guter Ausbeute erhalten. Die Selektivität, bezogen auf den Umsatz, liegt bei etwa 70 bis 90 %.

Das Verfahren liefert darüber hinaus ω-en-Carbonsäureester mit hoher Linearität, die meist bei über 90 % liegt. Als Nebenprodukt fällt ganz überwiegend ein ω-en-α-Methylcarbonsäureester an.

Die entständige Doppelbindung wird nicht isomerisiert. Es werden keine Produkte mit innenständiger Doppelbindung erhalten.

Das erfindungsgemäße Verfahren benötigt keine Edelmetallkatalysatoren, und es können einfache und in großen Mengen zugängliche Ausgangsverbindungen eingesetzt werden.

Beispiele

In den Beispielen, die die Erfindung verdeutlichen sollen, wird jeweils ein 5 l VA-Stahlautoklav mit Magnetrührer, Temperatur- und Druckregulierung verwendet. Die Reaktionskomponenten α,ω-Dien, Alkohol, Kobaltverbindung und Amin werden eingefüllt und auf Reaktionstemperatur gebracht.

Dann wird Wasserstoff aufgedrückt, worauf durch Aufdrücken von Kohlenmonoxid der Gesamtdruck aufgebaut wird. Der Druckabfall während der Reaktion wird durch ständige Kohlenmonoxid-Zufuhr ausgeglichen.

Nach beendeter Hydrocarboxyalkylierung wird auf Raumtemperatur abgekühlt und entspannt. Die Zusammensetzung des Reaktionsproduktes wird durch gaschromatographische Untersuchungen bestimmt. Die ω-en-Carbonsäure wird durch fraktionierte Destillation isoliert.

In den Beispielen sind %-Angaben jeweils Angaben in Gewichts-%.

Beispiel 1

Einwaage:

772,2 g ( 9,4 Mol) 1,5-Hexadien
451,8 g (14,1 Mol) Methanol
743,5 g ( 9,4 Mol) Pyridin
23,4 g ( 0,094 Mol) Co-(II)-acetat

Reaktionsbedingungen:

T = 140 °C, $P_{H2}$ = 10 bar (anfangs), $P_{ges}$ = 280 bar, t = 18 h

Bei einem Umsatz von 24,6 %, bezogen auf eingesetztes 1,5-Hexadien, werden erhalten:
16,8 % Hept-ω-ensäuremethylester
2,4 % Octandisäuredimethylester
5,4 % 2,5-Dimethylcyclopentanon

Beispiel 2

Einwaage:

870,6 g ( 7,9 Mol) 1,7-Octadien
632,8 g (19,8 Mol) Methanol
441,4 g ( 4,74 Mol) 4-Picolin
59,0 g ( 0,237 Mol) Co-(II)-acetat

Reaktionsbedingungen:

T = 140 °C, $P_{H2}$ = 10 bar (anfangs), $P_{ges}$ = 280 bar, t = 12 h

Bei einem Umsatz von 36,8 %, bezogen auf eingesetztes 1,7-Octadien, werden erhalten:
32,8 % Non-ω-ensäuremethylester
3,4 % Decandisäuredimethylester
0,6 % 2-Methyl-5-n-propylcyclopentanon

Beispiel 3

Einwaage:

914,7 g ( 8,3 Mol) 1,7-Octadien
664,8 g (20,8 Mol) Methanol
347,8 g ( 3,7 Mol) 4-Picolin
62,0 g ( 0,25 Mol) Co-(II)-acetat

Reaktionsbedingungen:

T = 140 °C, $P_{H2}$ = 10 bar (anfangs), $P_{ges}$ = 280 bar, t = 6 h

Bei einem Umsatz von 32,2 %, bezogen auf eingesetztes 1,7-Ocatdien, werden erhalten:
29,2 % Non-ω-ensäuremethylester
2,4 % Decandisäuredimethylester
0,5 % 2-Methyl-5-n-propylcyclopentanon

Beispiel 4

Es wird wie in Beispiel 3 verfahren. Die Reaktionszeit ist jedoch verlängert: t = 9 h.

Bei einem Umsatz von 39,7 %, bezogen auf eingesetztes 1,7-Octadien, werden erhalten:
34,6 % Non-ω-ensäuremethylester
4,1 % Decandisäuredimethylester
1,0 % 2-Methyl-5-n-propylcyclopentanon

Beispiel 5

Einwaage:

1 036,9 g ( 7,5 Mol) 1,9-Decadien
360,5 g (11,25 Mol) Methanol
593,3 g ( 7,5 Mol) Pyridin

18,7 g ( 0,075 Mol) Co-(II)-acetat

Reaktionsbedinungen:

T = 140 °C, $P_{H2}$ = 10 bar (anfangs), $P_{ges}$ = 280 bar, t = 18 h
Bei einem Umsatz von 37,6 %, bezogen auf eingesetztes 1,9-Decadien, werden erhalten:
31,6 % Undec-ω-ensäuremethylester
4,1 % Dodecandisäuredimethylester
1,2 % 2-Methyl-5-n-pentylcyclopentanon

Beispiel 6

Einwaage:

925,7 g ( 8,4 Mol) 1,7-Ocatdien
812,7 g (17,6 Mol) Ethanol
117,3 g ( 1,26 Mol) 4-Picolin
148,5 g ( 0,25 Mol) Co-(II)-naphthenat

Reaktionsbedingungen:

T = 130 °C, $P_{H2}$ = 5 bar (anfangs), $P_{ges}$ = 200 bar, t = 1 h
Bei einem Umsatz von 37,4 %, bezogen auf eingesetztes 1,7-Octadien, werden erhalten:
31,8 % Non-ω-ensäureethylester
4,4 % Decandisäurediethylester
1,2 % 2-Methyl-5-n-propylcylcopentanon

Beispiel 7

Einwaage:

683,2 g ( 6,2 Mol) 1,7-Octadien
1148,9 g (15,5 Mol) n-Butanol
73,6 g ( 0,93 Mol) Pyridin
109,6 g ( 0,19 Mol) Co-(II)-naphthenat

Reaktionsbedingungen:

T = 130 °C, $P_{H2}$ = 10 bar (anfangs), $P_{ges}$ = 280 bar, t = 1 h
Bei einem Umsatz von 43,7 %, bezogen auf eingesetztes 1,7-Octadien, werden erhalten:
35,9 % Non-ω-ensäurebutylester
5,9 % Decandisäuredibutylester
1,9 % 2-Methyl-5-n-propylcyclopentanon

## Ansprüche

1. Verfahren zur Herstellung von ω-en-Carbonsäureestern durch Hydrocarboxyalkylierung von α,ω-Dienen mit 5 bis 20 C-Atomen,
dadurch gekennzeichnet,
daß man die Reaktion mit Kohlenmonoxid in Gegenwart einer Kobaltverbindung und eines tertiären Amins in einem Alkohol bei 100 bis 200 °C und 150 bis 350 bar durchführt.

2. Verfahren nach Anspruch 1,
dadurch gekennzeichnet,
daß man als Kobaltverbindung Salze, Oxide und/oder Carbonyle des Kobalts einsetzt.

3. Verfahren nach Anspruch 1,
dadurch gekennzeichnet,
daß man als tertiäres Amin Pyridin und/oder nicht-ortho-substituierte Alkylpyridine verwendet.

4. Verfahren nach Anspruch 3,
dadurch gekennzeichnet,
daß Pyridin und/oder 4-Picolin verwendet wird.

5. Verfahren nach Anspruch 1,
dadurch gekennzeichnet,
daß man die Reaktion in einem Alkohol mit 1 bis 6 C-Atomen durchführt.

6. Verfahren nach Anspruch 1,
dadurch gekennzeichnet,
daß die Reaktion bei 120 bis 160 °C durchgeführt wird.

7. Verfahren nach Anspruch 1,
dadurch gekennzeichnet,
daß die Reaktion bei einem Druck von 200 bis 300 bar durchgeführt wird.

8. Verfahren nach Anspruch 1,
dadurch gekennzeichnet,
daß α,ω-Diene mit 8 bis 16 C-Atomen eingesetzt werden.

9. Verfahren nach Anspruch 1,
dadurch gekennzeichnet,
daß das Molverhältnis , -Dien : Kobaltverbindung 20 : 1 bis 200 : 1 beträgt.

10. Verfahren nach Anspruch 1,
dadurch gekennzeichnet,
daß das Molverhältnis tertiäres Amin : Kobaltverbindung 2 : 1 bis 300 : 1 beträgt.